# EUROPEAN PATENT APPLICATION

(11) **EP 2 763 065 A1**
(43) Date of publication of application: **06.08.2014**
(21) Application number: 13405010.3
(22) Date of filing: 31.01.2013
(51) Int. Cl.: G06F 19/00

(54) **Method and system for identifying a fluid sample**

(71) Applicant: Sensirion AG, 8712 Stäfa (CH)
(72) Inventor: Mayer, Felix, 8712 Stäfa (CH)
(74) Representative: Detken, Andreas

(57) **Abstract**

A portable electronic device (1) is equipped with a sensor that is sensitive to a plurality of chemical analytes. For identifying a fluid sample, the sensor is operated to obtain a measured variable. The measured variable is compared to reference variables in a database (3). Subject to a result of the comparison, identifiers associated with the reference variables are selected and presented to a user. In order to constantly improve the identification accuracy, the user provides feedback indicating whether the sample is correctly represented by at least one of the selected identifiers, and the database is modified subject to the user input.

## Description

### TECHNICAL FIELD

The present invention relates to a method for identifying a fluid sample (in particular, a gaseous sample) with a portable electronic device having a sensor that is sensitive to a plurality of analytes. The invention further relates to a corresponding system for identifying a fluid sample, to a correspondingly configured portable electronic device, and to corresponding computer program elements.

### BACKGROUND ART

Portable electronic devices such as mobile phones, tablet computers, notebook computers etc. have become ubiquitous in everyday life. Such devices are nowadays equipped with a multitude of sensors, including gyroscopes, acceleration sensors, magnetic field sensors, proximity sensors, cameras, GPS modules etc.

It would be desirable to integrate further sensors into portable electronic devices, in particular, sensors that are sensitive to chemical analytes. Such sensors will in the following be called "chemical sensors". It would be desirable to identify odors or gases with the aid of such sensors in a portable electronic device. For instance, it would be desirable to be able to position the portable electronic device near a particular fruit or a particular drink and to operate the portable electronic device to return the name of the fruit (e.g., "apple") or of the drink (e.g., "coffee"), or to expose the portable electronic device to a gas and to operate the portable electronic device to return the name and possibly concentration of the gas (e.g., "ozone"). In more general terms, it would be desirable to identify a particular fluid sample (usually a gaseous sample) to which the portable electronic device is exposed.

### SUMMARY OF THE INVENTION

In a first aspect, it is an object of the present invention to provide a method for identifying a fluid sample with a portable electronic device having a chemical sensor.

This problem is solved by a method having the features of claims 1. Further embodiments of the invention are laid down in the dependent claims.

The present invention provides a method for identifying a fluid sample with a portable electronic device having a first sensor, the first sensor being sensitive to a plurality of chemical analytes. The method comprises:
operating the first sensor in the portable electronic device to obtain a measured variable;
comparing the measured variable to reference variables in a database;
subject to a result of the comparison, selecting a set of identifiers associated with said reference variables;
operating the portable electronic device to present the selected identifiers to a user of the portable electronic device; and
operating the portable electronic device to receive user input (user feedback), the user input indicating whether the sample is correctly represented by at least one of the selected identifiers.

The method may further comprise:
modifying the database subject to the user input.

By modifying the database subject to the user feedback, a mechanism is provided for constantly improving the database quality and thus the identification accuracy while the portable electronic device is being used.

In the method, a measurement is carried out with a first sensor, which is a chemical sensor. The chemical sensor is sensitive to at least two different analytes, and preferably is sensitive to five or more different analytes. Chemical analytes to which the first sensor is sensitive can include a wide variety of chemical elements and compounds, including, without limitation: alcohols such as ethanol; organic acids such as formic acid and acetic acid; ketones such as acetone; aldehydes such as formaldehyde; carbon monoxide; carbon dioxide; ozone; ammonia; methane; benzene and benzene derivatives such as xylenes; thiols, in particular alkylthiols such as methyl mercaptan (methanethiol); and nitric oxides (NOx). The analyte will generally be present in a fluid medium, in particular, in a gas, more particularly in air.

The sample supplied to the chemical sensor may be analyzed by means of the chemical sensor as to which of the analytes that the chemical sensor is sensitive to are present in the sample, and possibly in what concentration. A combination of analytes detected in the gas supplied may suggest that a certain odor or a certain gas composition is present. The chemical sensor is capable of measuring one or more properties of multiple different analytes. Hence, the chemical sensor may be understood as a sensor device for detecting one or more properties of more than one analyte. A property may, for example, be a concentration of an analyte in a fluid which, for example, may be the air surrounding the device. Other properties may be, for example, chemical properties such as a binding energy of an analyte. It is noted that for the different analytes the chemical sensor is sensitive to it is not required to always measure the same property per analyte. Different properties may be measured for different analytes.

The first sensor may comprise one or more semiconductor sensor elements. These semiconductor sensor elements may comprise at least one sensitive layer, for which at least one electrical property (in particular, conductivity) changes in the presence of at least one chemical analyte due to adsorption and/or chemical reactions on the surface of the sensitive layer (including catalytic reactions in which the sensitive layer acts as a catalyst). The first sensor may include at least one heat source integrated within the sensor to heat the sensitive layer to an operating temperature thereof. In particular, the sensitive layer may be a metal oxide (MOX) layer. Sensors having at least one MOX layer as a sensitive layer will in the following be called MOX sensors. The metal oxide may be, e.g., tin oxide, tungsten oxide, gallium oxide, indium oxide, or zinc oxide. However, the present invention is not limited to MOX sensors. For instance, in other embodiments, the first sensor may function on an optical principle, i.e., an optical property of a sensor material may be modified such as its transmission rate, and this optical property is determined. Another possible measurement principle is a chemomechanical principle, in which a mass change upon absorption is transformed into a surface acoustic wave or into a cantilever resonance, for example.

The sensor may comprise two or more sensor elements ("cells") that have different sensitivities to selected analytes. The sensor cells may be arranged in a one- or two-dimensional array. Each sensor cell may provide a sensitive layer of a material exhibiting different sensitivity to some or all of the analytes that the sensor is sensitive to. For instance, each cell of the sensor array may specifically be mainly sensitive to a different analyte and as such may enable the portable electronic device to detect the presence or absence or concentration of such analyte. "Mainly" in this context shall mean that a sensor cell is more sensitive to the subject analyte than to other analytes. However, a sensor cell of such sensor array may exhibit not only sensitivity to its main analyte, but also to analytes other than the main analyte since such sensor cell may exhibit a cross-sensitivity to one or more analytes possibly representing main analytes for other cells. In this case, it is preferred that different sensor cells have different sensitivity profiles for the various analytes that the sensor is sensitive to. For instance, to discuss a particularly simple example, if one cell is sensitive to ethanol with a certain sensitivity and to acetone with a certain other sensitivity, it is preferred that another sensor cell is sensitive with a different ratio of sensitivities to ethanol and acetone, such that by comparing the signals of the two cells, the analytes ethanol and acetone can be separated.

The sensor cells may have different sensitivities to multiple different analytes at different operating conditions. For example, the sensor cell may mainly be receptive to a first analyte x when being heated to a first temperature Tx, and may mainly be receptive to a second analyte y when being heated to a second temperature Ty which is different from the first temperature Tx. To take advantage of this property, each of the sensor cells or specific groups of sensor cells may be provided with an individual heater. In other embodiments, all cells may be heated by the same heater. In some embodiments, the sensor may comprise only a single sensor cell that has different sensitivities to multiple different analytes at different operating conditions.

In case the chemical sensor comprises more than one sensor element or sensor cell, the individual sensor cells may be embodied as discrete sensor cells. The sensor cells are preferably mounted on a common conductor board of the portable electronic device. The sensor cells may take the form of multiple chips. Each individual chip may be packaged, i.e. encapsulated, separately. In an alternative arrangement, multiple or all chips may be packaged in a common package, such that these chips are encapsulated by a common encapsulation. In a further embodiment, multiple or all sensor cells are monolithically integrated in a common sensor chip with a common substrate for multiple or all sensor cells. Such a monolithic multiple sensor chip may still be encapsulated and be arranged on and electrically connected to a conductor board of the portable electronic device.

The results of a measurement with the chemical sensor are represented by a measured variable. The measured variable contains, in digitized (in particular, sampled) form, information derived from the sensor output. This information is dependent on the chemical composition of the sample. In particular, the measured variable may contain information related to the presence, absence and/or concentration of the various analytes that the sensor is sensitive to. In some embodiments, the measured variable may contain the immediate sensor output, e.g., the immediate output values of the individual sensor cells at one or more points in time, possibly together with the associated operating conditions at which these output values were obtained. In other embodiments, the measured variable may be the result of subjecting the direct sensor output to at least one pre-processing operation. The pre-processing operations may include, without limitation, one or more of the following: compensation algorithms, e.g. for compensating sensor drifts, including baseline correction operations; scaling or normalization (globally for all sensor cells or individually for each sensor cell); simple transforms such as nonlinear transforms and orthogonal transformations; more complex transforms such as discrete Fourier transforms (DFT) and discrete wavelet transforms (DWT); data reduction, including resampling; feature extraction, including calculation of baseline response, derivatives, integrals, fits to a model such as linear, polynomial and exponential fits, etc. The invention is not limited to any particular method of pre-processing. As a result of pre-processing, the measured variable need not necessarily directly correspond to the output of individual sensor cells, but may contain information that relates to the collective response of several sensor cells.

The measured variable may be supplied directly by the sensor, or the sensor may cooperate with a pre-processing module that receives raw sensor data from the sensor and derives the measured variable from the raw sensor data. The pre-processing module may be dedicated to the chemical sensor, or it may be embodied in software or firmware that is executed on a general-purpose processor of the portable electronic device.

The measured variable will generally be a data structure. The data structure may contain ancillary information in addition to the information that has been derived from the output of the chemical sensor. Elements of the data structure may represent, for example, one or more of the following: the unprocessed or pre-processed response of the sensor, as discussed above; associated operating conditions; information about the pre-processing algorithms that have been applied; one or more time stamps; information about the sensor (e.g., sensor model, serial number and calibration data stored with the sensor); information about the portable electronic device (e.g., the device's model, serial number and/or MAC address); information from other sensors integrated in the portable electronic device, such as a humidity sensor and/or a temperature sensor; geolocation (including GPS) information referring to the location of the portable electronic device at the time of measurement; and any other kind of ancillary information. With such additional information, the sample identification request may be better tracked and data processing may be improved.

In particular, the measured variable may be supplemented with ancillary information that is representative of at least one parameter that is different from the chemical composition of the sample. To this end, the portable electronic device may comprise a second sensor, the second sensor being sensitive to a parameter that is different from the chemical composition of the sample, and the method may comprise:
operating the second sensor in the portable electronic device; and
associating ancillary information derived from signals of the second sensor with the measured variable.

If the second sensor is a temperature sensor or a humidity sensor, output from the second sensor may help in compensating temperature induced and/or humidity induced signal variations in a signal of the chemical sensor. Preferably the temperature sensor and/or the humidity sensor may be arranged in proximity to the chemical sensor, for example in the same opening of a housing of the portable electronic device. However, the second sensor may be another type of sensor, such as a pressure sensor, an inertial sensor, a light sensor, a camera, a microphone etc. It is also possible that all or part of the ancillary information is supplied to the portable electronic device by the user by operating an input device of the portable electronic device, e.g., by typing in a parameter via a keyboard.

In some embodiments, the measured variable may comprise a measurement tuple (which can also be referred to as a vector or array) having a set of tuple elements, wherein each tuple element is assigned to a dedicated cell of the chemical sensor and/or to a dedicated operating condition of the chemical sensor or of a cell of the chemical sensor. In the measurement tuple, after a measurement, each tuple element may hold a value measured with respect to the assigned sensor cell and/or the assigned operating condition during a measurement. In those cases where each sensor cell and/or each operating condition is embodied for being mainly sensitive to an analyte assigned, each measurement tuple may be interpreted as an indicator of the presence and possibly a concentration of such analyte.

The measured variable is compared to reference variables in a database. The reference variables can have the same data structure as the measured variable, or they can have a different data structure. For instance, the reference variables may contain only part of the information contained in the measured variable, they may contain additional information, they may information in a different form, and/or they may contain a different type of information. It is therefore envisaged to optionally subject the measured variable and/or the reference variables to initial data treatment before comparison. The initial data treatment may comprise, for example, any kind of data treatment that has been mentioned above in connection with pre-processing.

Each reference variable may represent a reference composition, i.e., a real or virtual measurement result for a particular chemical composition. Many other ways how reference variables in the database relate to particular chemical compositions are conceivable, and the invention is not restricted to any particular manner in which reference data are represented in the database, or to any particular manner in which the database is organized. The reference variables may be associated with additional information, such as a reliability indicator (see below) and/or one or more sample categories.

As a result of the comparison between the (possibly processed) measured variable and the (possibly processed) reference variables, a set of identifiers associated with the reference variables are retrieved from the database. Preferably, each identifier represents a particular chemical composition. For instance, if each of the reference variables represents a particular reference composition, exactly one identifier may be associated with each reference variable, and the identifier may be a tag for that reference composition. The identifier may be a verbal descriptor. For example, if the reference variable represents a composition of analytes that is characteristic for the smell of apples, the identifier might be the word "apple". In other embodiments, the identifier may be embodied as a numeral identifier or as any other kind of identifier that is suitable for classifying chemical compositions. Depending on the database structure, each identifier may be uniquely associated with a single reference variable, or each identifier may be associated with a plurality of ("similar") reference variables. The invention is not restricted as to the manner in which reference variables and identifiers are related to each other.

The reference variables and identifiers in the database may initially be provided by a database provider who may have measured various reference samples by means of a portable electronic device containing the same kind of chemical sensor as is used for measuring the actual samples, and who has created the associated identifiers. For example, the provider may have held the portable electronic device close to a reference sample (e.g., an apple), have triggered a measurement, have had the measurement result stored in form of a reference variable, and have created a descriptor for the reference sample ("apple") as an identifier. The reference variable and the associated identifier may have been transmitted to the database, e.g. via a wireless link in case the portable electronic device has a wireless communication capability.

Preferably, the retrieved identifiers represent those chemical compositions that are most likely to correspond to the measured variable (the "closest matches"). For determining the "distance" between the measured variable and any particular reference variable, a suitable norm may be employed. The definition of the norm will strongly depend on the exact data structures of the reference variables and of the measured variables and to the kind of data treatment that is applied to these variables. Comparison between the measured variable and the reference variables may involve any known extraction or pattern recognition method, including the use of neural networks, statistical analyses, cluster analyses etc. The present invention is not restricted to a particular manner in which the comparison is carried out. If no close matches are found, an empty set of identifiers or an empty (void) identifier may be returned, e.g., the descriptor "no close matches" or a numerical identifier that represents such a descriptor.

The selected identifiers may be complemented with quality indicators or likelihood values indicating a likelihood that a particular identifier actually represents the chemical composition assigned to the identifier. The likelihood values can be regarded as indicators of the quality of agreement between the measured variable and one or more reference variables. For instance, the likelihood value could be inversely related to the norm value of the distance between the measured variable and the "closest" reference variable. For example, if the measurement aimed at identifying a fruit by its odor, and if the closest match was a reference variable for an apple while more "distant" matches were reference variables for a pear and an apricot, the selected identifiers might be descriptors like "apple", "pear", "apricot", and each identifier might be complemented by a likelihood value in the following manner: "apple: likelihood 80/100", "pear: likelihood 34/100", "apricot: likelihood 18/100".

Concretely, in some embodiments, the reference elements may be reference tuples with each reference tuple representing a particular sample. An identifier for the sample represented by the concerned reference tuple may be associated with each reference tuple. The elements of a given reference tuple may be assigned to dedicated cells of the chemical sensor and/or dedicated operating conditions of the chemical sensor or of the cell of the chemical sensor, which may correspond to the various analytes the chemical sensor mainly is sensitive to. Each tuple element may then hold a value characteristic for being sensed by the dedicated cell of the chemical sensor and/or under the dedicated operating condition of the chemical sensor or of the cell of the chemical sensor in the presence of the sample represented by the concerned reference tuple. Such a value may preferably be a normalized value. Hence, a reference tuple represents a sample such as an odor and/or gas and specifically defines a sample with respect to the set of analytes the chemical sensor is sensitive to. For example, if the chemical sensor is mainly sensitive to nine different analytes, a reference tuple representing the odor of a "tulip" may contain nine tuple elements with each tuple element providing a value reflecting the presence, absence, or partial presence of the assigned analyte out of the nine analytes the chemical sensor mainly is sensitive to. Each reference tuple may then be tagged by an identifier identifying the odor and/or gas which is represented by the subject reference tuple. The comparison between measurement tuples and reference tuples may include a determination of a deviation between each measurement tuple element and its counterpart reference tuple element. In this context, a measurement tuple element and its counterpart reference tuple element may be linked in that they are assigned to the same cell of the chemical sensor and/or to the same operating condition of the chemical sensor or of the cell of the chemical sensor. Hence, both tuple elements would indicate a value, an in particular a normalized value, of e.g. the absence, presence or partial presence of the assigned analyte which is measured on the one hand, and which is provided within the reference tuple for characterizing the presence of the assigned analyte in the subject odor on the other hand. Since a tuple generally contains at least two tuple elements, there may be at least two deviations determined in the evaluation process. In case normalization is applied to the values of the measurement and reference tuple elements, at least three tuple elements are required for allowing comparison of at least two of the tuple elements of the measurement and the reference tuple since one of the tuple elements is used for normalization. Hence, it may depend on a result of the at least two deviations which identifier is returned. For example, each deviation may be compared to an assigned threshold, and the identifier of the reference tuple under investigation is returned in case a number n of deviations is below the assigned thresholds. Here, the norm used for comparing measurement tuples and reference tuples would be the number n of analytes that closely match a typical concentration of the subject analytes as is manifested in the subject reference tuple for the subject sample.

The selected identifiers are presented to a user of the portable electronic device. For instance, the identifiers and possibly the associated likelihood values may be shown on a display of the portable electronic device, in verbal or graphical form, or they may be outputted in acoustic form through a loudspeaker or through earphones.

The portable electronic device then waits to receive user input (user feedback) whether one of the presented identifiers correctly represents the measured sample. User feedback may be inputted, e.g., via a touchscreen of the portable electronic device by the user tapping onto the correct identifier, via a keyboard of the portable electronic device, via voice recognition, or by any other suitable means.

If an empty (void) set or an empty identifier was retrieved, or if none of the retrieved identifiers correctly represents the measured sample, the user may be allowed create a new identifier for the sample. To this end, the user may be allowed to provide (e.g., type) a descriptor for the sample. For instance, if the selected identifiers were "apple", "pear", and "apricot", but the measured sample was gas emanating from a peach, the user might type in the descriptor "peach". The portable electronic device may then transmit the new descriptor to the database for a search whether the new descriptor ("peach") matches any identifier that is already present in the database or whether any such identifier is similar to the descriptor, and to present, as a result, a new set of suggested identifiers to the user for selection. For instance, if the user had typed in the descriptor "peach", and if the identifier "peach" was found in the database, the portable electronic device may confirm to the user that the identifier "peach" has been found and has been assigned to the measured variable. If, for example, the user had misspelled the word "peach" as "peech", the search for matching or similar identifiers might still return the identifier "peach", and this identifier would be presented to the user for confirmation.

The user feedback may then be used to modify the database. In other words, the database may be "trained" by the user. This can take place in a wide variety of different manners. For instance, the database may be modified by modifying one or more existing reference variables, and/or by adding a new reference variable based on the measured variable to the database, wherein the identifier that has been confirmed by the user to be correct is associated with the new reference variable. If none of the retrieved identifiers correctly represents the sample, or if the retrieved set of identifiers is void or only a void identifier was returned, the database may be modified by adding a new reference variable to the database based on the measured variable and associating a new identifier with the new reference variable.

In some embodiments, a reliability indicator might be assigned to each reference variable in the database. The reliability indicator of one or more reference variables might then be modified subject to the user feedback. In particular, the reliability indicator may initially have a comparatively low value for new reference variables created by user feedback while having a higher value for reference variables that had been provided by the database provider or that have otherwise been confirmed to be reliable. The initially assigned value for new reference variables may depend on various factors, such as the likelihood value that has been described above. For instance, if the likelihood value is high, the reliability indicator may initially be set to a higher value than if the likelihood value is low. The reliability indicator may be modified during subsequent measurements. For instance, if in subsequent measurements the same or a different user consistently obtains measured variables for the same type of sample that are close to the new reference variable, this may be used to increase the value of the reliability indicator. If, on the other hand, later measurements consistently show that the new reference variable is far away from subsequently measured variables for the same type of sample, the reliability indicator may be decreased. The reliability indicator may be used to purge the database of unwanted entries, e.g., by deleting those reference variables that are associated with the lowest reliability indicators.

Preferably before a measurement with the chemical sensor is carried out, or before the measured variable is compared to the reference variables, the user may be prompted to supply a sample category. To this end, the user may be prompted to select a sample category from a list, or the user may be prompted to supply a descriptor for the sample category to be compared to sample categories already present in the database. The sample category can then be used to restrict the search space in the database to a subset of reference variables, i.e., the measured variable would be compared only to reference variables that are associated with the supplied sample category. The sample category can also be used to subject the measured variable to a particular pre-processing operation that depends on the sample category. In this manner, more reliable results can be obtained.

It is within the scope of the present invention to allow the user to create new database entries or even entire new sample categories. To this end, the method may comprise:
i. optionally, prompting the user to supply a sample category;
ii. prompting the user to supply a descriptor representative of a new reference sample;
iii. operating at least the first sensor in the portable electronic device to obtain a measured variable representative of the new reference sample;
iv. optionally, repeating steps ii.-iv. for one or more further new reference samples to obtain descriptors and associated measured variables representative of a plurality of new reference samples; and
v. adding at least one of the following items to the database: new reference identifiers based on descriptors that were supplied in step ii., associated new reference variables based on measured variables that were obtained in step iv., and sample categories that were supplied in step i.

The database can be configured to interact with an evaluation unit that carries out some of the steps of the above-described methods. Several possibilities exist for where the database and the evaluation unit may be located. The evaluation unit may of course carry out many further tasks.

According to a first possibility, both the database and the evaluation unit are arranged remote from the portable electronic device. In view of the database claiming considerable storage space, a server / a server system / a cloud system may preferably be used for providing the database. The evaluation unit may be represented by processing power at the same system remote from the portable electronic device or on a different system remote from the portable electronic device. A service provider may offer services to users or subscribers for identifying samples received in form of measured variables. A user may register with the provider and receive the identifiers in return for a measured variable sent. Any transmission of the measured variables and/or of the returned identifiers may be carried out via a wireless link, e.g. based on a UMTS or a GPRS standard.

According to a second possibility, the database is remote from the portable electronic device, however, the evaluation unit is arranged in the portable electronic device. The evaluation unit may be embodied as hardware or firmware or as software on a general purpose processor unit of the portable electronic device. In this scenario, the electronic portable device may request one or more reference variables from the database for locally comparing these to the measured variable on the portable electronic device. Again, a wireless link may be used for requesting/fetching and receiving reference variables from the database.

In both these possibilities, more than one user may have access to the database. User feedback from a plurality of users may therefore be employed to constantly expand the amount and quality of reference data in the database and to thereby improve identification accuracy for all users.

According to a third possibility, both the database and the evaluation unit are arranged in the portable electronic device. Given that the storage space in portable electronic devices such as in smartphones has considerably grown over the previous years, a local storage of the entire database may be envisaged. In this case, the chemical sensor, the evaluation unit and the database all reside in the portable electronic device. Identifiers are returned within the portable electronic device and may be displayed on a display of the portable electronic device. The database may be updated regularly by a service provider, as may be the evaluation algorithm, and data from the database may be regularly transmitted to the service provider to update a master database.

In particular if the evaluation unit and the database are arranged remote from the portable electronic device, the method may comprise:
sending the measured variable from the portable electronic device to the evaluation unit;
operating the evaluation unit to compare the measured variable to the reference variables in the database;
returning the selected identifiers from the evaluation unit to the portable electronic device;
sending data that depends on the user input from the portable electronic device to the evaluation unit and/or the database.

The method may further comprise modifying the database subject to the sent data.

The portable electronic device may be any of the following: a mobile phone, and in particular a smart phone, a handheld computer, an electronic reader, a tablet computer, a game controller, a pointing device, a photo or a video camera, or a computer peripheral. This listing is to be understood as not limiting. Such portable electronic device may primarily be designed for computing and/or telecommunication and/or other tasks in the IT arena, and may be enhanced by the function of providing chemical information as to its environment. The user may learn about chemical substances, compositions and/or odors present in the device's surroundings, and may use, transmit or else further analyze such information. Since such portable electronic devices typically include interfaces to a remote infrastructure, such information may also be transmitted elsewhere and used elsewhere. In an alternative, the user himself/herself may benefit from the information provided by the chemical sensor in that actions may be taken in response to detected sample, including but not limited to toxic substances and compounds such as CO. An alarm may be triggered once such compound is identified in form of a return of the "CO" identifier, for example. Other measurements may be taken out of pure interest, such as, for example, the measurement of the odor of a flower or the odor of a drink. In response to such measurement, the identifier/name of an odor unknown to the user, such as "sunflower", for example, or "bourbon 10 yrs old", may be displayed to the user.

In another aspect, the present invention provides a system for identifying a sample, the system comprising:
a portable electronic device having an input device, an output device and a first sensor, the first sensor being sensitive to a plurality of chemical analytes, the portable electronic device being configured to operate the first sensor to obtain a measured variable;
a database containing reference variables and associated identifiers; and
an evaluation unit for comparing the measured variable to reference variables in the database, the evaluation unit being configured to select a set of identifiers subject to a result of the comparison,
wherein the portable electronic device is configured to operate the output device to present the selected identifiers to a user of the portable electronic device (i.e., wherein the portable electronic device has a presentation module for operating the output device to present the selected identifiers to a user of the portable electronic device); and
wherein the portable electronic device is configured to operate the input device to receive user input indicating whether the sample is correctly represented by at least one of the selected identifiers (i.e., wherein the portable electronic device has a feedback-receiving module for operating the input device to receive user input indicating whether the sample is correctly represented by at least one of the selected identifiers).

The portable electronic device may further be configured to supply feedback data that depends on the user input to the evaluation unit and/or the database so as to modify the database (i.e., the portable electronic device may have a database modification module for supplying the feedback data to the evaluation unit and/or the database so as to modify the database).

The input device may be, e.g., a keyboard, a touchscreen or a microphone. The output device may be, e.g., a display, a loudspeaker etc. These lists are not limiting. For each task to be carried out by the portable electronic device, by the evaluation unit and the database, a corresponding module may be provided. These modules may be implemented fully or partially in software and/or firmware.

As explained above, the database and the evaluation unit may be arranged remote from the portable electronic device. They are preferably connectable to the portable electronic device via a wireless link. The portable electronic device may then be configured to send the measured variable to the evaluation unit. The evaluation unit may be configured to return the selected identifiers to the portable electronic device. The portable electronic device may be configured to send feedback data that depends on the user input to the evaluation unit and/or to the database. The database may be configured to be modified subject to the sent data. To this end, the evaluation unit may be configured to modify the database.

The portable electronic device may be equipped with a second sensor, the second sensor being sensitive to a parameter that is different from the chemical composition of the sample. The portable electronic device may then be configured to operate the second sensor and to associate ancillary information derived from signals of the second sensor with the measured variable.

In a further aspect, the present invention provides a portable electronic device for use in a method for identifying a sample, the portable electronic device comprising:
an input device;
an output device; and
a first sensor, the first sensor being sensitive to a plurality of chemical analytes,
wherein the portable electronic device is configured to operate the first sensor to obtain a measured variable,
wherein the portable electronic device is configured to supply the measured variable to an evaluation unit for comparing the measured variable to reference variables in a database, the evaluation unit being configured to select a set of identifiers subject to a result of the comparison,
wherein the portable electronic device is configured to operate the output device to present the selected identifiers to a user of the portable electronic device, and
wherein the portable electronic device is configured to operate the input device to receive user input indicating whether the sample is correctly represented by at least one of the selected identifiers

The portable electronic device may further be configured to supply, to the evaluation unit and/or the database, feedback data that depends on the user input so as to modify the database.

For each task that the portable electronic device is configured to carry out, a corresponding module may be provided. These modules may be implemented fully or partially in software and/or firmware.

In yet another aspect, the present invention provides computer program code elements that carry out central parts of the method described above when executed in a processor. Each computer program element comprises computer-implemented instructions to cause a processor to carry out a particular method. It can be provided in any suitable form, including source code or object code. In particular, it can be stored on a computer-readable medium or embodied in a data stream. The data stream may be accessible through a network such as the Internet.

In particular, the invention provides a computer program element comprising computer code that, when executed in a processor of a portable electronic device, carries out the following steps:
operating a first sensor of the portable electronic device, the first sensor being sensitive to a plurality of chemical analytes, to obtain a measured variable;
supplying the measured variable to an evaluation unit;
receiving a set of identifiers from the evaluation unit;
operating an output device of the portable electronic device to present the identifiers to a user of the portable electronic device; and
operating an input device of the portable electronic device to receive user input indicating whether the sample is correctly represented by at least one of the presented identifiers.

The computer program element may further comprise computer code that, when executed in a processor of a portable electronic device, carries out the following step:
supplying data that depends on the user input to the evaluation unit and/or to an associated database containing reference variables so as to modify the database.

The invention further provides a computer program element comprising computer code that, when executed in a processor of an evaluation unit connected to a database, carries out the following steps:
receiving, from a portable electronic device, a measured variable that contains information about a chemical composition of a sample;
comparing the measured variable to reference variables in the database;
selecting a subset of reference variables subject to a result of the comparison;
for each reference variable of said subset, selecting an identifier associated with said reference variable;
returning the selected identifiers from the evaluation unit to the portable electronic device;
receiving, from the portable electronic device, feedback data (representing user input, the user input indicating whether the sample is correctly represented by at least one of the selected identifiers); and
modifying the database subject to the feedback data.

All considerations that are discussed in this document in conjunction with the method of the present invention are equally applicable to the system, to the portable electronic device and to the computer program elements of the present invention.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows a usage scenario with a mobile phone;
- Fig. 2: shows a highly schematic block diagram of a mobile phone;
- Fig. 3: shows a functional diagram of a sample identification system;
- Fig. 4: shows a highly schematic illustration of a chemical sensor chip;
- Fig. 5: shows an illustration of a comparison step in a sample identification method;
- Fig. 6: shows an illustration of two different measurement tuples;
- Fig. 7: shows a flow diagram of a sample identification method;
- Fig. 8: illustrates a possible user dialog for a sample identification method;
- Fig. 9: shows a flow diagram for a method of creating new reference variables; and
- Fig. 10: illustrates a possible user dialog for a method of creating new reference variables.

### DESCRIPTION OF PREFERRED EMBODIMENTS

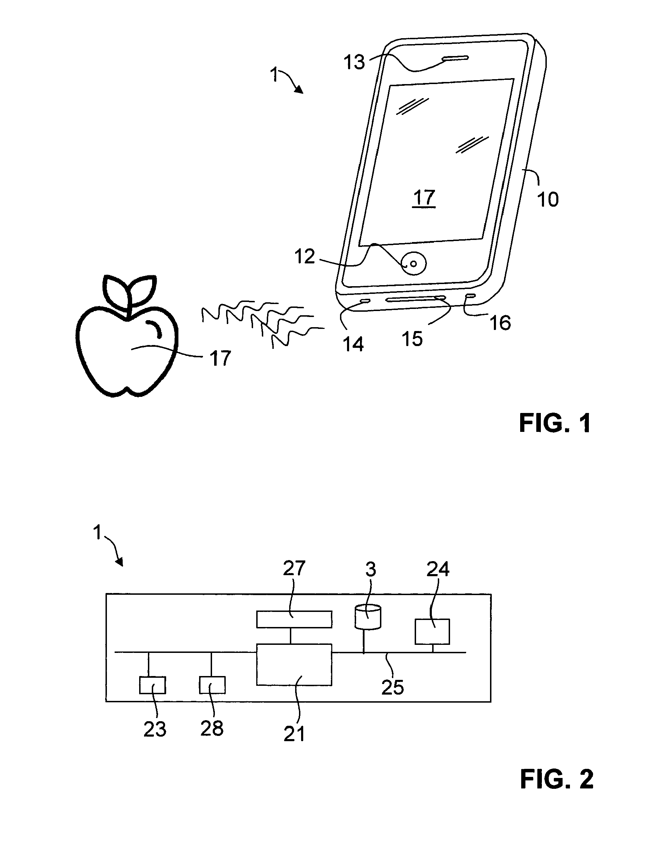

Figure 1 illustrates a usage scenario with a portable electronic device in the form of a mobile phone 1. The mobile phone has a housing 10, an input/output device in the form of a touchscreen display 11 and a further input device in the form of a pushbutton 12. Below a first opening 13 in the front of the housing 10, an output device in the form of a loudspeaker is arranged. In a lower sidewall region of the housing 10, further openings 14, 15 and 16 are provided. Behind these openings, components such as a microphone, further loudspeakers and connectors are disposed. In addition, behind any of these openings sensors such as a humidity sensor, a temperature sensor and one or more sensors for detecting at least one chemical analyte (i.e., one or more chemical sensors) may be arranged. The chemical sensor may comprise one or more sensor cells, each sensor cell exhibiting a different sensitivity to selected analytes. The mobile phone runs an application program (app) for identifying samples. The present example, the app is configured to carry out identification of gas samples of the odors of different fruits. The mobile phone 1 is held close to a an apple 17, which emits gaseous chemical substances providing an apple-specific odor. The chemical substances are sensed by the chemical sensor. By means of a database and an evaluation unit - which are, in this example, arranged in the mobile phone 1 itself - a measured variable that represents the sensed odor is compared to reference variables that represent known fruit odors. By this comparison, the measured variable is finally identified as corresponding to the odor from an apple. This result is displayed on the display 11 of the mobile phone 1, and the user is asked for confirmation. Depending on whether or not the user confirms that the mobile phone has correctly identified the object to be an apple, the database is then modified.

Figure 2 shows a schematic hardware-oriented block diagram of the mobile phone 1. A microprocessor 21 is connected via electrical conductors 22 to a chemical sensor 23. The chemical sensor 23 contains signal processing capability in order to output a measured variable. A routine for analyzing the measured variable supplied by the chemical sensor 23 may be executed by an evaluation unit. A hardware of the evaluation unit may be represented by the microprocessor 21, and a software of the evaluation unit may be represented by a program element stored in a memory 24 connected to the microprocessor 21 via a bus system 25. A database 3 containing reference variables may be integrated in the mobile phone 1 and may be connected to the microprocessor 21 via the bus system 25. The database 25 may be resident in the same physical memory device as the memory 24 or in a separate memory, e.g., in a dedicated memory card inserted into the mobile phone 1. A wireless interface 27 and at least one further sensor 28 (e.g., a humidity sensor, a temperature sensor, an inertial sensor etc.) may be connected to the microprocessor 21. Input/output devices as previously mentioned may further be connected to the microprocessor 21.

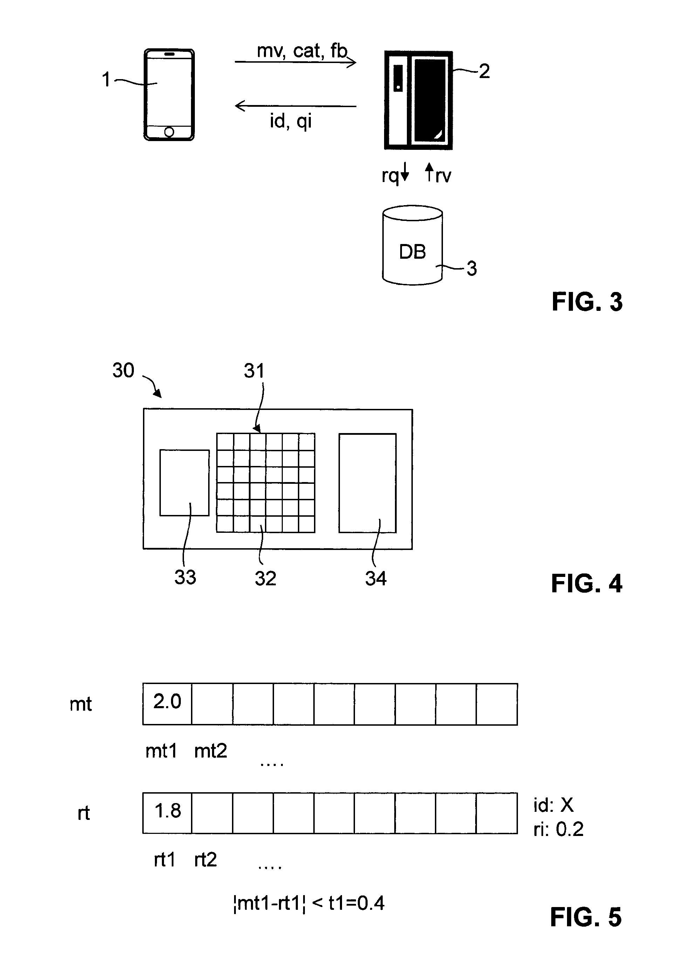

Figure 3 illustrates a functional diagram of a sample identification system, wherein an evaluation unit 2 and a database 3 are arranged remote from the portable electronic device. The portable electronic device again takes the form of a mobile phone 1 and includes a chemical sensor. The chemical sensor provides a measured variable mv to the evaluation unit 2, optionally together with a sample category cat that has previously been selected by the user. The evaluation unit 2 may be embodied by a server hosting a service for sample identification. Once the evaluation unit 2 has received a measured variable mv and optionally the sample category cat, this is taken as a request to start an identification process. This process is initiated by submitting a request rq to a database 3 for reference variable data. The database 3 contains storage space for storing reference variables rv representing various samples. The reference variables rv are transmitted to the evaluation unit 2 as requested and are compared to the measurement variable mv there. Once a close match is identified between the measurement variable mv and one of the reference variables rv, an identifier id for the sample (e.g., the name of an odor or gas) represented by this specific reference variable rv is returned to the mobile phone 1, together with a quality indicator (likelihood value) qi that indicates the deviation between the measured variable mv and the reference variable rv. The identifier id and the quality indicator qi are displayed on the display of the mobile phone 1, and the user is asked for confirmation. Depending on whether or not the user confirms that the system has correctly identified the odor or gas, the mobile phone sends a feedback message fb to the evaluation unit, which then modifies the database accordingly.

Figure 4 illustrates a highly schematic view of a chip 30 comprising a chemical sensor as it may be used in conjunction with the present invention. The chip 30 comprises a chemical sensor structure 31 which takes the form of a sensor array comprising multiple symbolic sensor cells 32, in the present example, six times six sensor cells 32. In addition a humidity sensitive structure 33 is arranged next to the chemical sensor structure 32, and electronic circuitry 34 is integrated into the chemical sensor chip 30, which electronic circuitry 34 is responsible for linearizing and A/D converting the sensor signal, for applying a humidity compensation of the signal, and for outputting a measured variable.

Figure 5 illustrates a possible embodiment of a comparison step in a sample identification method. In this embodiment, the measured variable takes the form of a measurement tuple mt. Each element mt1, mt2, ... of the measurement tuple corresponds to the (possibly pre-processed, in particular, humidity-compensated) signal from one of the sensor cells 32. For illustration purposes, only the value 2.0 of the first measurement tuple element mt1 is shown. The reference variable in this embodiment takes the same form as the measured variable, i.e., it takes the form of a reference tuple rt having the same number of tuple elements as the measurement tuple mt, complemented by an identifier id=X and a reliability indicator ri=0.2. For illustration purposes, only the value 1.8 of the first reference tuple element rt1 is shown. The reference tuple rt represents a particular odor that is identified by the identifier id=X. The first measurement tuple element mt1 is assigned to a first sensor cell of the chemical sensor which first sensor cell is mainly sensitive to a first analyte. The corresponding first reference tuple element rt1 is assigned to the same sensor cell and hence, provides an expected value as to the same first analyte for the odor with identifier id=X. The value of the first measurement tuple element mt1 is compared to the first reference tuple element rt1 by determining a deviation |mt1-rt1|, which deviation is compared to a first threshold t1, which threshold is the present example is set to 0.4. The interpretation of the foregoing may be as follows: A concentration of 2.0 of the first analyte was measured. The reference odor named "X" typically shows a concentration of this analyte of 1.8. As long as the deviation of the measured concentration from the reference concentration is less than 0.4, the measured odor and/or gas may be "X" subject to a concentration of the other analytes measured which concentrations may be compared to the reference concentrations in the same way. If a match is finally determined, the reliability indicator gives an indication how much reliability can be assigned to the given reference tuple rt. For instance, if this particular reference tuple was supplied only recently by an unknown user, a low reliability value might be assigned to the reference tuple. The reliability indicator may be employed by the evaluation unit for taking a decision whether or not to continue to compare the measurement tuple mt to further reference tuples.

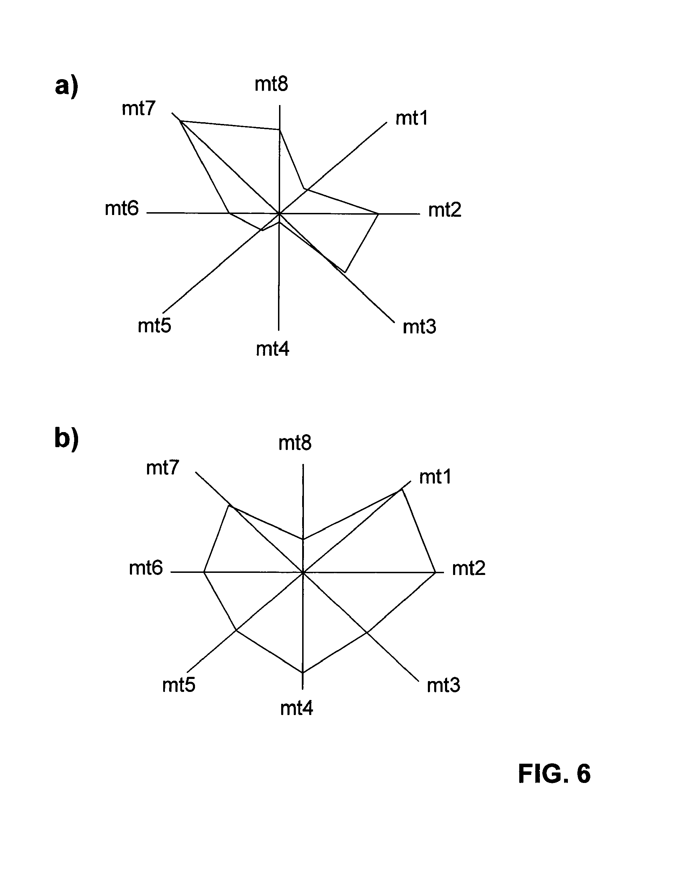

Figure 6 illustrates in diagrams a) and b) two different possible measurement tuples mt. Each measurement tuple mt is sensed by a chemical sensor being sensitive to eight analytes, such that a measurement tuple mt contains eight tuple elements mt1 ... mt8. The measurement tuple mt according to part a) represents a measurement of a first gas/odor, and the measurement tuple mt according to part b) represents a measurement of a second gas/odor. Each measurement tuple mt is represented by a spider diagram with eight axes, and with the value of each tuple element mt1 ... mt8 being reflected on the respective axis crossing. The values as shown may be normalized values in order for a specific odor to result in the same spider diagram every time independent of a strength of the odor. For example, each measured value may be converted into a normalized value by dividing the value by the value measured for the first sensor cell, i.e. mt1. Preferably, normalized patterns are compared to each other. Other approaches of normalizations ma be applied, for example, a normalization with respect to an area of the spider diagram, etc. Such spider diagram measurement tuple representation may support graphical pattern recognition. The reference tuples may also be stored as graphical representations in form of spider diagrams. In a best match algorithm, the reference pattern that matches the measurement pattern best is identified and its tag is returned.

Of course, many other possibilities for comparing measured variables to reference variables exist, including sophisticated methods of statistical analysis, neural networks etc.

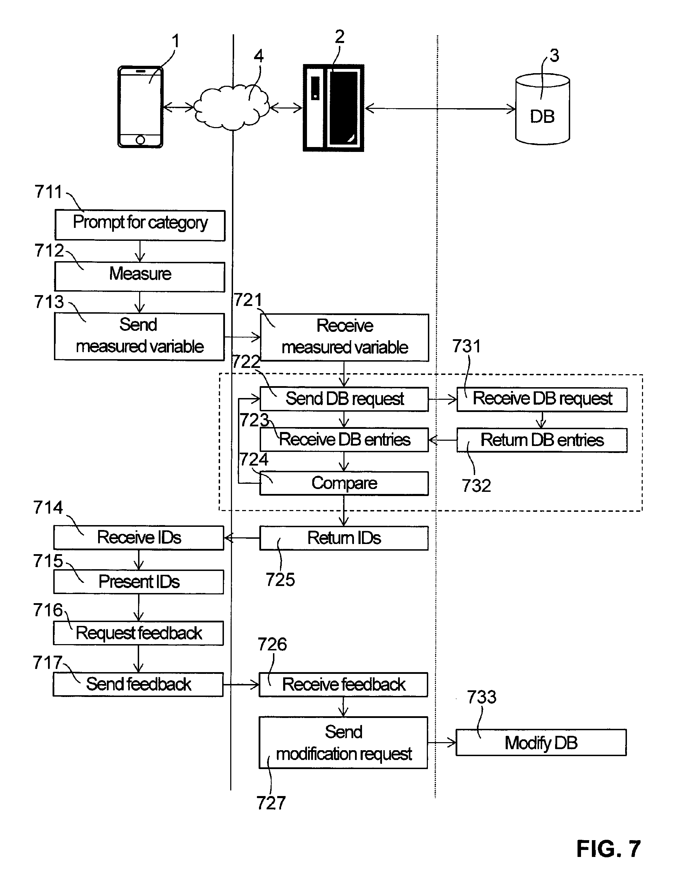

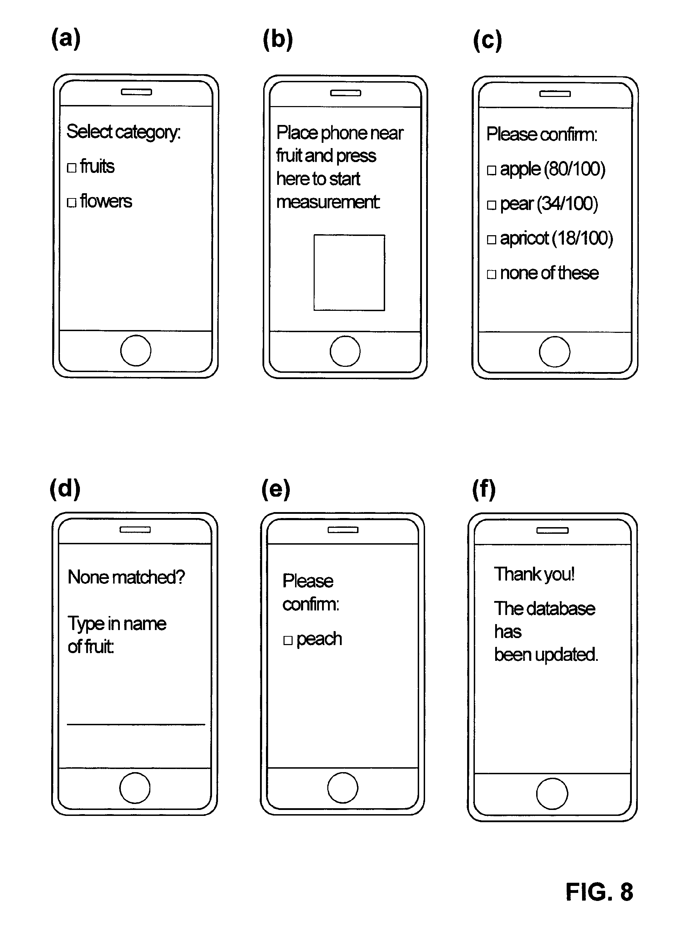

Figure 7 illustrates a flow diagram of a method for sample identification, in which the database is modified subject to user feedback. The method is carried out by computer programs which run, on the one hand, on a mobile phone 1 and, on the other hand, on a remote evaluation unit 2 and a database 3 connected to the evaluation unit 2. As will become apparent in the following, these computer programs cooperate by exchanging data via a network, in particular, a wireless network 4. The computer program (app) which runs on the mobile phone guides the user through the process by displaying messages on the touchscreen of the mobile phone and receiving user input through the touchscreen. Fig. 8 illustrates a possible user dialog carried.

In step 711, the app on the mobile phone 1 prompts the user to supply a sample category to the mobile phone 1. A possible screen dialog on the touchscreen display of the mobile phone is illustrated in Figure 8 (a). For instance, the app on the mobile phone may ask the user whether he wishes to carry out the identification of "fruits" or of "flowers". The user selects a category by tapping on to one of the category descriptors. In the present example, the user selects "fruits".

In the subsequent step 712, the mobile phone carries out a measurement, employing the chemical sensor and possibly additional sensors. To this end, the user dialog requests the user to place the phone near the object to be measured and to press a button in order to start the measurement, an example being shown in Figure 8 (b). Based on the sensor signals obtained during the measurement, the mobile phone constructs a measured variable.

The mobile phone then sends the measured variable to the evaluation unit 2 in step 713. The evaluation unit 2 receives the measured variable in step 721 and creates the first of a sequence of database requests in step 722. The database 3 receives the database requests in step 731 and returns one or more database entries in the form of reference variables in step 732. The evaluation unit 2 receives the reference variables in step 723 and compares the reference variables to the measured variable in step 724. The evaluation unit repeats the sequence of database requests and comparison steps until one or more reference variables have matched the measured variable according to certain predefined criteria.

In step 725, the evaluation unit looks up and selects the identifiers for the matching reference variables in the database and returns them to the mobile phone 1. Optionally, the evaluation unit associates a quality indicator or likelihood value to each identifier. The mobile phone receives the identifiers and optionally the likelihood values in step 714 and presents them to the user in step 715. The mobile phone requests feedback from the user whether one or more of the identifiers are correct (step 716). An example is shown in Figure 8 (c). In this example, the evaluation unit has selected three identifiers ("apple", "pear", "apricot") together with associated likelihood values (80/100, 34/100 and 18/100). The mobile phone presents these identifiers and likelihood values to the user and asks the user to select one of the identifiers. If the user thinks that none of the identifiers matches the fruit that he wishes to identify, he can select a further option "none of these". In this particular example, the user has taken a measurement on a peach, which is not in the list of identifiers presented by the mobile phone. The user would therefore tap onto the selector "none of these". The mobile phone would in return ask the user to provide the name of the fruit. An example for this part of the user dialog is shown in Figure 8 (d). The user would now type in the word (descriptor) "peach". The mobile phone would send the new descriptor "peach" to the evaluation unit for a search in the database whether this descriptor is similar to existing identifiers. If the identifier "peach" is found in the database, it is sent back to the mobile phone and presented to the user, as exemplified in Figure 8 (e). The user is now asked to confirm whether this identifier is correct. The mobile phone now transmits this information as user feedback to the evaluation unit in step 717.

The evaluation unit receives the feedback in step 726 and creates a database modification request based on the feedback. It sends the modification request to the database in step 727. The database receives the modification request in step 733, and the database entries are modified accordingly. At the same time, the user is informed that his feedback has been used to modify the database (Fig. 8 (f)). In particular, the evaluation unit may modify the database by adding a new reference variable based on the measured variable that was supplied to the evaluation unit in step 721, and associating the new reference variable with the existing identifier "peach". The new reference variable may be complemented by a reliability indicator that reflects that the new reference variable has been added to the database as a result of user feedback (see below).

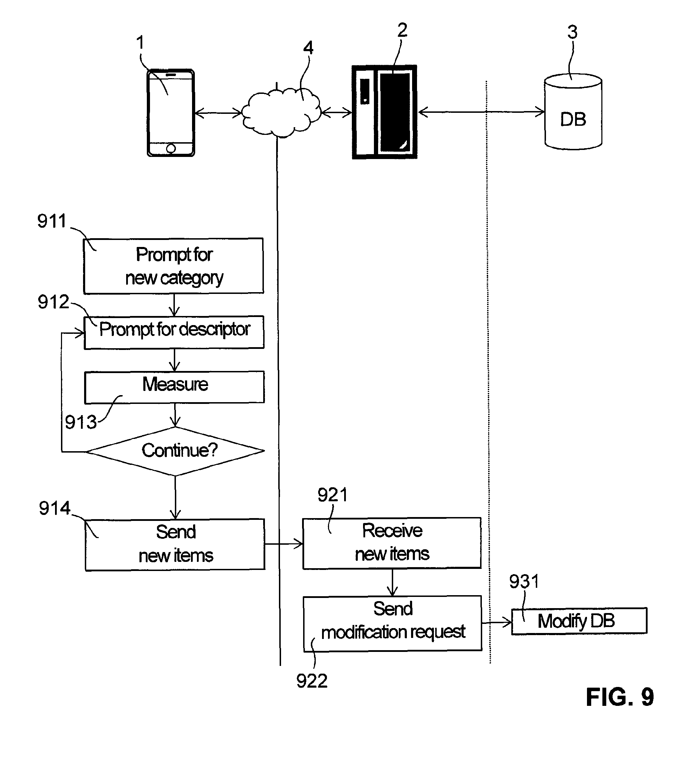

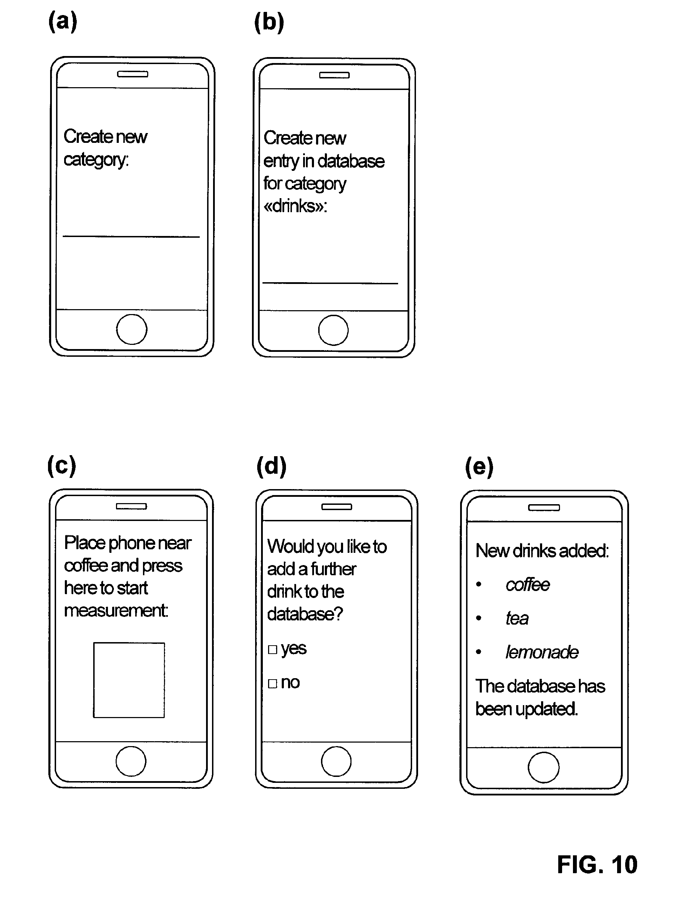

Optionally, the app on the mobile phone allows the user to create new categories, and the program running on the evaluation unit allows such new categories to be added to the database. This is illustrated in Figures 9 and 10. In step 911, the app on the mobile phone prompts the user to supply the name of the new category. In the present example, the user would type in the category name "drinks". An example of the user dialog is shown in Figure 10 (a).

In step 912, the app then prompts the user to supply a descriptor for a new entry in the database within the category "drinks" (Fig. 10 (b)). In the present example, the user might type in the descriptor "coffee". The mobile phone might now create a new identifier for this descriptor, or it might send the descriptor to the evaluation unit to search in the database for identifiers that are similar to the descriptor, so as to be able to ask the user whether any existing identifier matches the new sample. In this manner it can be avoided that multiple identifiers are created for identical sample types that are already represented in the database. Once an identifier has been created or selected based on the descriptor that had been supplied by the user in step 912, the app on the mobile phone will now direct the user to carry out a measurement for the corresponding sample. A possible corresponding user dialog is shown in Figure 10 (c). The mobile phone carries out a measurement in step 913 and stores the resulting measured variable in a memory of the mobile phone. This procedure is repeated a number of time until measurements have been taken for all desired samples in the new category "drinks".

The mobile phone then sends the new category name, all measured variables and the associated identifiers to the evaluation unit in step 914. The evaluation unit receives these new items. It derives new reference variables from the measured variables and sends a modification request to the database in step 922. As a result, the new reference variables and their corresponding identifiers are stored in the database, together with the associated category name.

In order to ensure that newly created reference variables are not given the same credibility as reference variables that had been created, e.g., by the database provider, each reference variables may be associated with a credibility or reliability indicator. The value of the reliability indicator would be lower for newly created reference variables than for reference variables that had been provided by the database provider. In the present example, the newly created reference variable associated with the identifier "coffee" would be assigned an initial reliability value of, say, 0.2, whereas reference variables which have been provided by the database provider and which have been validated by many subsequent measurements would be assigned a reliability value of, say, 1.0. If subsequent measurements by the same or other users consistently confirm that the new reference variable is representative for the drink "coffee", this information might be used by the evaluation unit to increase the value of the reliability indicator for the corresponding reference variable. If, on the other hand, subsequent measurements by the same or other users show that the measured variables for coffee are consistently very different from the new reference variable, the reliability indicator might be further decreased. The database might periodically be purged of reference variables whose reliability indicator is below a certain threshold, for example below 0.1.

New categories may be provided with a flag for review by a human operator, and this flag may be reset once a human operator has reviewed and validated the new category or has possibly reassigned a new category name, has merged the new category with an existing category, or has otherwise modified the new category.

While there are shown and described presently preferred embodiments of the invention, it is to be understood that the invention is not limited thereto but may be otherwise variously embodied and practised within the scope of the following claims.

## Claims

1. A method for identifying a fluid sample with a portable electronic device (1) having a first sensor (23), the first sensor being sensitive to a plurality of chemical analytes, the method comprising:
operating the first sensor (23) in the portable electronic device (1) to obtain a measured variable (mv);
comparing the measured variable (mv) to reference variables (rv) in a database (3);
subject to a result of the comparison, selecting a set of identifiers (id) associated with said reference variables (rv);
operating the portable electronic device (1) to present the selected identifiers (id) to a user of the portable electronic device (1);
operating the portable electronic device (1) to receive user input, the user input indicating whether the sample is correctly represented by at least one of the selected identifiers (id).

2. The method of claim 1, further comprising:
modifying the database (3) subject to the user input.

3. The method of claim 2, wherein the database (3) is modified by at least one of the following:
modifying at least one of the reference variables (rv) in the database;
adding at least one new reference variable (rv) to the database;
modifying a reliability indicator (ri) associated with at least one of the reference variables (rv) in the database.

4. The method of claim 2 or 3, wherein the user is allowed to create a new identifier if a void identifier is selected or if none of the selected identifiers (id) correctly represents the sample, and wherein the database (3) is modified by adding a new reference variable to the database (3) based on the measured variable (mv) and associating the new identifier with the new reference variable.

5. The method of any one of the preceding claims, comprising:
before comparing the measured variable (mv) to the set of reference variables (rv), prompting the user to supply a sample category (cat); and
comparing the measured variable (mv) only to those reference variables (rv) that belong to the supplied sample category (cat).

6. The method of any one of the preceding claims, wherein the database (3) interacts with an evaluation unit (2), wherein the database (3) and the evaluation unit (2) are arranged remote from the portable electronic device (1) and are preferably connected to the portable electronic device (1) via a wireless link (4), and wherein the method comprises:
sending the measured variable (mv) from the portable electronic device (1) to the evaluation unit (2);
operating the evaluation unit (2) to compare the measured variable (mv) to the reference variables (rv) in the database (3);
returning the selected identifiers (id) from the evaluation unit (2) to the portable electronic device (1);
sending feedback data (fb) that depends on the user input from the portable electronic device (1) to the evaluation unit (2) and/or database (3).

7. The method of any one of the preceding claims, wherein the measured variable (mv) is associated with ancillary information representative of at least one parameter that is different from the chemical composition of the sample.

8. The method of any one of the preceding claims, the method further comprising:
i. optionally, prompting the user to supply a sample category;
ii. prompting the user to supply a descriptor representative of a new reference sample;
iii. operating at least the first sensor in the portable electronic device to obtain a measured variable representative of the new reference sample;
iv. optionally, repeating steps ii.-iv. for one or more further new reference samples to obtain descriptors and associated measured variables representative of a plurality of new reference samples; and
v. adding at least one of the following items to the database: new reference identifiers based on descriptors that were supplied in step ii., associated new reference variables based on measured variables that were obtained in step iv., and sample categories that were supplied in step i.

9. A system for identifying a sample, the system comprising:
a portable electronic device (1) having an input device, an output device and a first sensor (23), the first sensor being sensitive to a plurality of chemical analytes, the portable electronic device (1) being configured to operate the first sensor (23) to obtain a measured variable (mv);
a database (3) containing reference variables (rv) and associated identifiers (id); and
an evaluation unit (2) for comparing the measured variable (mv) to reference variables (rv) in the database, the evaluation unit (2) being configured to select a set of identifiers (id) subject to a result of the comparison,
wherein the portable electronic device (1) is configured to operate the output device to present the selected identifiers (id) to a user of the portable electronic device (1); and
wherein the portable electronic device (1) is configured to operate the input device to receive user input indicating whether the sample is correctly represented by at least one of the selected identifiers (id).

10. The system of claim 9, wherein the portable electronic device (1) is configured to supply, to the evaluation unit (2) and/or the database (3), feedback data (fb) that depends on the user input so as to modify the database (3), and wherein the database (3) is configured to be modified subject to the feedback data (fb).

11. The system of claim 9 or 10,
wherein the database (3) and the evaluation unit (2) are arranged remote from the portable electronic device (1) and are preferably connectable to the portable electronic device via a wireless link;
wherein the portable electronic device (1) is configured to send the measured variable (mv) to the evaluation unit (2);
wherein the evaluation unit (2) is configured to return the selected identifiers (id) to the portable electronic device (1);
wherein the portable electronic device (1) is configured to send feedback data that depends on the user input to the evaluation unit (2) and/or to the database (3).

12. The system of any one of claims 9-11,
wherein the portable electronic device (1) has a second sensor (28), the second sensor being sensitive to a parameter that is different from the chemical composition of the sample; and
wherein the portable electronic device (1) is configured to operate the second sensor (28) and to associate ancillary information derived from signals of the second sensor (28) with the measured variable.

13. A portable electronic device (1) for use in a method for identifying a sample, the portable electronic device comprising:
an input device;
an output device; and
a first sensor (23), the first sensor being sensitive to a plurality of chemical analytes,
wherein the portable electronic device (1) is configured to operate the first sensor (23) to obtain a measured variable (mv),
wherein the portable electronic device (1) is configured to supply the measured variable (1) to an evaluation unit (2) for comparing the measured variable to reference variables (rv) in a database (3), the evaluation unit (2) being configured to select a set of identifiers (id) subject to a result of the comparison,
wherein the portable electronic device (1) is configured to operate the output device to present the selected identifiers (id) to a user of the portable electronic device (1), and
wherein the portable electronic device (1) is configured to operate the input device to receive user input indicating whether the sample is correctly represented by at least one of the selected identifiers (id).

14. The portable electronic device of claim 13, wherein the portable electronic device (1) is configured to supply, to the evaluation unit (2) and/or the database (3), feedback data that depends on the user input so as to modify the database (3).

15. A computer program element comprising computer code that, when executed in a processor of a portable electronic device (1), carries out the following steps:
operating a first sensor (23) of the portable electronic device (1), the first sensor (23) being sensitive to a plurality of chemical analytes, to obtain a measured variable (mv);
supplying the measured variable (mv) to an evaluation unit (2);
receiving a set of identifiers (id) from the evaluation unit (2);
operating an output device of the portable electronic device (1) to present the identifiers (id) to a user of the portable electronic device (1);
operating an input device of the portable electronic device (1) to receive user input indicating whether the sample is correctly represented by at least one of the presented identifiers (id).

16. A computer program element comprising computer code that, when executed in a processor of an evaluation unit (2) connected to a database (3), carries out the following steps:
receiving, from a portable electronic device (1), a measured variable (mv) that contains information about a chemical composition of a sample;
comparing the measured variable (mv) to reference variables (rv) in the database (3);
subject to a result of the comparison, selecting a set of identifiers (id) associated with said reference variables (rv);
returning the selected identifiers (id) from the evaluation unit (2) to the portable electronic device;
receiving, from the portable electronic device (1), feedback data (fb); and
modifying the database (3) subject to the feedback data.
